# EUROPEAN PATENT APPLICATION

(11) **EP 4 286 399 A1**
(43) Date of publication of application: **06.12.2023**
(21) Application number: 22177134.8
(22) Date of filing: 03.06.2022
(51) Int. Cl.: C07K 5/062, C07C 319/20, C07C 321/14, C07D 241/08

(54) **RECOVERY OF METHIONYLMETHIONINE FROM AQUEOUS ALKALI METAL IONS CONTAINING MEDIA**

(71) Applicant: Evonik Operations GmbH, 45128 Essen (DE)
(72) Inventor: MUSIC, Arif, 63450 Hanau (DE); BRAUNE, Sascha, 69221 Dossenheim (DE); BÄRZ, Manfred, 63579 Freigericht (DE); WEFER, Johannes, 098412 Singapore (DE); JÄGER, Barbara, 63533 Mainhausen (DE)
(74) Representative: Evonik Patent Association

(57) **Abstract**

The present invention concerns the recovery of methionylmethionine from aqueous alkali ions containing media by intermediate formation of Bis(methionyl)-diketopiperazine.

## Description

The present invention concerns the recovery of methionylmethionine from aqueous alkali metal ions containing media by intermediate formation of Bis(methionyl)-diketopiperazine.

Methionine is an essential amino acid, i.e., an amino acid which must be supplied in the course of the livestock production to accelerate the growth of the animals. The dipeptide DL-methionyl-DL-methionine (also referred to as "methionylmethionine" or "MetMet" in the following; sold as AQUAVI^{®}MetMet by Evonik Operations, Germany), comprising two chemically linked methionine units, has the advantage in comparison to methionine itself that due to its lower water solubility it exhibits a slow and/or retarded release effect, which makes it in particular suitable as feed ingredient in aquaculture applications, where leaching of monomeric amino acids is normally a problem, as for example documented in WO 2010/043558. But the slow and/or retarded release effect is also of benefit in the feeding of classical livestocks like poultry and swine.

MetMet can be produced from methionine hydantoin, which is supplied from the methionine process, and alkali base to yield intermediary 2,6-Bis(methionyl)-1,4-diketopiperazine (also referred to as "DLLD/DDLL-Met-DKP" or "Met-DKP" in the following). After purification, this Met-DKP can be hydrolyzed under basic conditions and then neutralized with sulfuric acid to yield the desired MetMet according to formula (I) (see also WO2010043558).

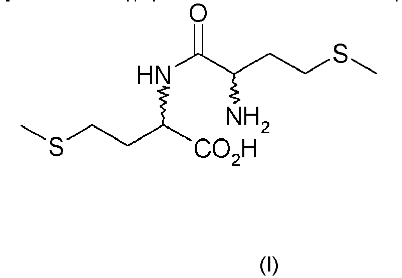

But as big amounts of a side-product are created in the chemical production of methionine, which contain a significant amount of MetMet, the question arose, if MetMet contained in such a side-product could not be utilized to increase the overall efficiency of the methionine production process.

Methionine is produced chemically for example via the Bucherer-Bergs reaction, which is a variant of the Strecker synthesis. Here, the starting substances 3- methylmercaptopropanal (prepared from 2-propenal and methylmercaptan), hydrocyanic acid (hydrogen cyanide), ammonia and carbon dioxide are reacted to give 5-(2- methylmercaptoethyl)hydantoin (methionine hydantoin) and this substance is subsequently hydrolysed with potassium carbonate and potassium hydrogencarbonate to give potassium methioninate. Methionine is finally liberated from its potassium salt by treatment with carbon dioxide, which may be filtered off as a precipitate from the mother liquor containing potassium carbonate and potassium hydrogencarbonate. The ammonia, potassium carbonate and potassium hydrogencarbonate reagents and also carbon dioxide are generally recycled in industrial methionine production. Instead of the mentioned potassium compounds also the corresponding sodium compounds can be used in the reaction. However, it is necessary from time to time to partially exchange the aqueous mother liquor of this hydantoin hydrolysis circulation for fresh potassium hydroxide or fresh sodium hydroxide, essentially to remove ("purge") inactivated potassium or sodium salt from the circulation in the form of neutral potassium or sodium formate, wherein the potassium or sodium formate is formed from the residues of hydrogen cyanide and potassium or sodium salts from the hydantoin hydrolysis present in the methionine hydantoin solution.

The so-called purge solution thus obtained normally contains besides high amounts of potassium or sodium salts (generally about 6 to 14 wt.-% potassium in form of potassium salts), methionine in amount of approximately 2 to 6 wt.-% and MetMet in an amount of approximately 2 to 8 wt.-%. The water content of this side-product is normally about 70 to 80 wt.-%. Due to the potassium, nitrogen and sulphur content, this side-product is suitable as liquid fertilizer. It would be desirable, however, to be able to recover the MetMet as contained in the side-product, increasing the overall efficiency of the process.

Attempts to utilize MetMet from this side-product directly failed up to date, because classical isolation methods like crystallization lead to a product with insufficient purity. A particular problem in isolating the MetMet from the side-product are the substances which are contained in the side-product in substantial amounts, in particular alkali metal ions like potassium, but also hydrogencarbonate. In addition, also organic compounds which are present in the side-product turned out to be an obstacle for the direct isolation of MetMet from the medium in pure form.

Thus, it was an object of the present invention to provide a method for enabling the utilization of MetMet from such a side-product or from other MetMet and potassium and optionally further disturbing substances containing aqueous media, wherein a particular object was to recover MetMet from such media in sufficiently pure form.

Surprisingly, it was found out that the object of the present invention could be solved by converting MetMet as contained in the aqueous solution into water-insoluble methionine diketopiperazine (3,6-Bis[2-(methylthio)ethyl]-2,5-piperazinedione; "Met-DKP") according to formula (II)

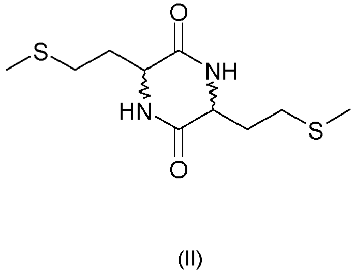

The water-insoluble Met-DKP can then easily be crystallized and isolated from the aqueous solution.

Thus, a first subject of the present invention is a method of recovery of MetMet from an aqueous alkali metal ions containing medium characterized in that MetMet as contained in the medium is converted into Met-DKP.

According to the invention, the conversion of MetMet into Met-DKP is preferably carried out by heating the medium to a temperature of 120 to 200 °C, in particular 150 to 180 °C, preferably 155 to 175 °C, more preferably 160 to 170 °C. The reaction is preferably carried out in a pressure vessel, in particular in an autoclave, so that due to the heating a pressure is generated, wherein the pressure during the reaction is preferably from 2 to 12 bar, more preferably from 4 to 10 bar, in particular from 5 to 7 bar. The reaction is preferably carried out for 0.5 to 10 hours, more preferably for 1 to 6 hours, in particular for 2 to 5 hours.

The reaction worked best at a pH of between 3 and 7. Thus, according to the invention, the medium has preferably a pH, measured with a pH electrode at 20°C, of 3 to 7, more preferably 3.5 to 6, in particular 4.0 to 5.5.

As the alkali metal ions containing side-product of the chemical methionine production process has normally an alkaline pH, in particular a pH, measured with a pH electrode at 20°C, of 7.5 to 12.5, more preferably of 8 to 10, the method of the invention preferably comprises as further, preceding step the acidification of such a starting medium, wherein by acidification preferably a pH of 3 to 7, in particular 3.5 to 6, more preferably 4.0 to 5.5, is adjusted.

Acidification is preferably carried out by using an acid selected from phosphoric acid, acetic acid, formic acid, sulfuric acid or mixtures thereof.

The alkali metal ions containing aqueous medium, from which the MetMet is recovered, is preferably a complex matrix, in particular a by-product of a chemical methionine production process.

The alkali metal ions which are contained in the aqueous medium are preferably selected from potassium ions, sodium ions and mixtures thereof, but are more preferably potassium ions.

The aqueous medium is preferably an aqueous solution, but can also be an aqueous suspension.

The aqueous medium as used in the process preferably has a water content of 50 to 90 wt.-%, more preferably of 60 to 85 wt.-%, in particular of 70 to 80 wt.-%, i.e., it has preferably a total dry matter content of 10 to 50 wt.-%, more preferably of 15 to 40 wt.-%, in particular of 20 to 30 wt.-%.

The aqueous medium, as used in the process, preferably contains MetMet, in relation to the total dry matter content of the aqueous medium, in an amount of 5 to 20 wt.-%, preferably in an amount of 8 to 16 wt.-%. Overall, the aqueous medium preferably contains MetMet in an amount of 1 to 12 wt.-%, preferably in an amount of 2 to 8 wt.-%, more preferably in an amount of 3 to 7 wt.-%.

The aqueous medium further contains alkali metal ions, preferably potassium or sodium ions, above all potassium ions, in particular, in relation to the total dry matter content of the aqueous medium, 10 to 50 wt.-%, more preferably 15 to 40 wt.-%, above all 20 to 35 wt.-%, alkali metal ions, which are present in the medium in the form of alkali metal salts. Overall, the aqueous medium preferably contains alkali metal ions, in the form of alkali metal salts, in an amount of at least 1 or at least 2 wt.-%, in particular in an amount of 2 to 18 wt.-%, more preferably in an amount of at least 4 wt.-%, in particular in an amount of 4 to 16 wt.-%, above all in an amount of at least 6 wt.-%, in particular in an amount of 6 to 14 wt.-%.

The aqueous medium preferably further contains hydrogencarbonate, in particular, in relation to the total dry matter content of the aqueous medium, 5 to 40 wt.-%, more preferably 10 to 35 wt.-%, above all 15 to 30 wt.-% hydrogencarbonate. Overall, the aqueous medium preferably contains hydrogen carbonate in an amount of at least 1 wt.-%, in particular in an amount of 1 to 15 wt.-%, more preferably in an amount of at least 2 wt.-%, in particular in an amount of 2 to 12 wt.-%, above all in an amount of at least 3 wt.-%, in particular in an amount of 3 to 10 wt.-%.

The aqueous medium further preferably also contains methionine, preferably in an amount of 1 to 10 wt.-%, preferably in an amount of 2 to 6 wt.-%.

The amounts of the organic substances as mentioned before are preferably determined by HPLC, in particular by HPLC as disclosed more in detail in the working examples. The amount of potassium is preferably determined by IC (ion chromatography), in particular by IC as disclosed more in detail in the working examples. The amount of hydrogencarbonate is preferably determined by titration with hydrogen chloride.

A typical methionine side stream medium, which is used for utilizing MetMet, preferably has a pH value of 7.5 to 12.5, more preferably of 8 to 10. Acidification of the medium preferably takes place by slow addition of acids such as phosphoric acid, acetic acid, formic acid or sulfuric acid (30%-96%), under constant stirring until a pH of 3 to 7, preferably 3.5 to 6 is reached, wherein very preferably sulfuric acid is used for acidifying the medium. Typically, foaming and increase of temperature are observed, which depend on the acid source used.

As next step, preferably after 5 to 60 minutes, more preferably after 5 to 30 minutes, the reaction matrix is preferably transferred into an autoclave and, after sealing the reaction mixture, it is preferably constantly stirred and heated to 150 to 180 °C, preferably 160 to 170 °C. Upon reaching the desired internal temperature, the reaction mixture is preferably stirred for further 0.5 to 3 hours, more preferably 1 to 2 hours, before being cooled, preferably to a temperature of about 70 °C. Crystallization of the Met-DKP is preferably already initiated by lowering the pressure in the reaction vessel. The resulting slurry is preferably filtered and the obtained solid may be washed with distilled water. The solid can then directly be used for recovering MetMet. Alternatively, the solid can be dried.

In one preferred embodiment of the invention, the method comprises the step of first removing a big part, preferably the major part (i.e. at least 50 %), of the alkali metal ions from the medium, before converting MetMet into Met-DKP. This is preferably done by using sulfuric acid for acidification of the alkaline starting medium and adjusting a pH value of between 3 to 7, in particular 4 to 6, preferably 4 to 5, which leads to precipitation of alkali metal ions as contained in the medium in form of alkali metal sulfate. The alkali metal sulfate thus obtained can then be separated by filtration or decantation. By doing so, preferably at least 50 wt.-% of the alkali metal ions as previously contained in the medium are removed. As next step, the pH can optionally be lowered further, before heating the medium under pressure as disclosed before to allow the formation of Met-DKP. The advantage of this method is that Met-DKP can be directly obtained in very pure form due to the previous removal of disturbing alkali metal ions, so that the alkali metal ions or salts thereof will not disturb in the formation of the Met-DKP precipitate.

In an alternative embodiment of the invention, no intermediary step for removing alkali metal ions as contained in the aqueous medium is employed, but the medium as obtained after acidification is directly employed in the pressure vessel for producing Met-DKP. This can lead to the formation of a Met-DKP precipitate which contains a higher amount of alkali metal salts like alkali metal sulfate. But surprisingly, it was found out that the alkali metal sulfate as contained in the Met-DKP precipitate can rather easily be separated by washing with water or with an aqueous solution. Thus, also this alternative method is commercially feasible for obtaining sufficiently pure Met-DKP.

In a further preferred embodiment of the invention, an acid is used for acidification of the alkaline starting medium which forms alkali metal salts with higher water solubility, like acetic acid, formic acid or phosphoric acid. The advantage of this method is that the alkali metal salt, or at least the major part thereof, stays in soluble form and thus Met-DKP can be obtained directly in relatively pure form and high yield, without the need of an intermediary separation of alkali metal ions and also without the need of a subsequent elaborate purification step.

The water-insoluble Met-DKP, as formed in the reaction, can easily be crystallized or precipitated and isolated from the aqueous medium, preferably by filtration, centrifugation or decantation, wherein crystallization or precipitation are preferably carried out at a temperature of from 0 to 140°C, more preferably at a temperature of 20 to 100°C. Subsequently, the Met-DKP as obtained might be purified further, preferably by washing with water or an aqueous solution and/or by recrystallization.

As next step, the Met-DKP thus obtained can be utilized in different ways like by re-converting it into MetMet again, in particular by recycling Met-DKP into the MetMet process, or by hydrolyzing it further to obtain methionine.

Conversion of Met-DKP to MetMet can be carried out by acidic or basic hydrolysis.

The acidic hydrolysis is preferably carried out in the presence of an acid which is selected from the group of mineral acids, HCI, H₂CO₃, CO₂/H₂O, H₂SO₄, phosphoric acids, carboxylic acids and hydroxy carboxylic acids.

The basic hydrolysis is preferably carried out at a pH of from 7 to 14, in particular at a pH of from 9 to 12, above all at a pH of from 10 to 11, in order to obtain DL-methionyl-DL-methionine. It is moreover possible for the basic conditions to be adjusted by using a substance which is preferably selected from the group of nitrogen-containing bases, NH₄HCO₃, (NH₄)₂CO₃, NH₄OH/CO₂ mixture, carbamate salts, KHCO₃, K₂CO₃, carbonates, alkali metal and alkaline earth metal bases.

The acidic or basic hydrolysis is preferably carried out at temperatures of from 50°C to 200°C, preferably from 80°C to 180°C and particularly preferably from 90°C to 160°C. To avoid conversion of Met-DKP into methionine, the acid or base is preferably added in an about equal molar amount, i.e. 1 to 1.2 mol acid or base equivalents to 1 mol of Met-DKP. Further, the residual amount of Met-DKP is preferably detected analytically and the reaction is stopped by cooling, when no further Met-DKP can be detected in the aqueous medium.

### Working Examples

### Example 1: Acidification of methionine side stream solution by pH shift with sulfuric acid

100 g of methionine side stream solution (3.2 wt.-% Met) containing 3.7 wt.-% MetMet (1.9 wt.-% DDLL-MetMet, 1.8 wt.-% DLLD-MetMet) with a measured pH value of 9.9 and a total K-content of 97.4 g/kg is acidified by slow addition of conc. sulfuric acid (11.6 g, 96%) at room temperature under constant stirring until pH 4 is reached. A typical foaming behavior and increase in temperature to around 40 °C is observed. After 10 minutes, the resulting precipitate is collected, which per IC analysis is characterized as potassium sulphate. The remaining mother liquor (84.5 g) is used for further transformation in example 3.

### Example 2: Acidification of methionine side stream solution with sulfuric acid for improved precipitation of potassium sulphate

500 g of methionine side stream solution (total K-content 103.2 g/kg) with a measured pH value of 9.4 is acidified by slow addition of conc. sulfuric acid (53.6 g, 96%) at room temperature under constant stirring until pH 5 is reached. A typical foaming behavior and increase in temperature to around 45 °C is observed. The suspension is then cooled to 10 °C and after 15 minutes, the resulting precipitate is collected, which per IC analysis is characterized as potassium sulphate. The remaining mother liquor can be used for further transformations as described in the following examples.

### Example 3: Synthesis and isolation of Met-DKP from methionine side stream solution

84.5 g of the remaining mother liquor from example 1 are transferred into a suitable autoclave and after sealing the reaction mixture is constantly stirred and heated to 160 °C. Upon reaching the desired internal temperature (around 6 bar pressure), the reaction is stirred for further 2.5 hours, before being cooled to 70 °C within 1 hour. The resulting slurry is filtered and the obtained solid washed with 25.0 g of distilled water. The solid is dried over night at 50 °C to yield 2.2 g of Met-DKP in 52% yield (82% purity).

### Example 4: Acidification of methionine side stream solution by pH shift with acetic acid and isolation of Met-DKP

100 g of methionine side stream solution (3.0 wt.-% Met) containing 3.3 wt.-% MetMet (1.6 wt.-% DDLL-MetMet, 1.7 wt.-% DLLD-MetMet) with a measured pH value of 8.3 and a total K-content of 93.1 g/kg is acidified by slow addition of glacial acetic acid (30.8 g, 99%) under constant stirring at room temperature until pH 5 is reached. Only mild foaming behavior, no increase in temperature and no precipitation are observed. After 5 minutes, 123.5 g of the solution are transferred into a suitable autoclave and after sealing the reaction mixture is constantly stirred and heated to 160 °C. Upon reaching the desired internal temperature (around 6 bar pressure), the reaction is stirred for further 2.5 hours, before being cooled to 70 °C within 1 hour. The resulting slurry is filtered and the obtained solid washed with 24.8 g of distilled water. The solid is dried over night at 50 °C to yield 1.5 g of Met-DKP in 46% yield (96% purity).

### Example 5: Acidification of methionine side stream solution by pH shift with sulfuric acid and isolation of Met-DKP

100 g of methionine side stream solution (3.0 wt.-% Met) containing 3.3 wt.-% MetMet (1.6 wt.-% DDLL-MetMet, 1.7 wt.-% DLLD-MetMet) with a measured pH value of 8.3 and a total K-content of 93.1 g/kg is acidified by slow addition of conc. sulfuric acid (8.7 g, 96%) under constant stirring at room temperature until pH 5.5 is reached. A typical foaming behavior and increase in temperature to 38 °C is observed. After 10 minutes, 100.1 g of the resulting slurry are directly transferred into a suitable autoclave and after sealing the reaction mixture is constantly stirred and heated to 160 °C. Upon reaching the desired internal temperature (around 6 bar pressure), the reaction is stirred for further 2.5 hours, before being cooled to 70 °C within 40 minutes. The resulting slurry is filtered and the obtained solid washed with 27.4 g of distilled water. The solid is dried over night at 50 °C to yield 5.7 g of Met-DKP in 87% yield (45% purity).

### Example 6: Acidification of concentrated and methionine reduced side stream solution by pH shift with sulfuric acid and isolation of Met-DKP

100 g of methionine reduced side stream solution (2.1 wt.-% Met) containing 6.5 wt.-% MetMet (3.4 wt.-% DDLL-MetMet, 3.1 wt.-% DLLD-MetMet), with a measured pH value of 8.8 and a total K-content of 130.0 g/kg is acidified by slow addition of conc. sulfuric acid (11.3 g, 96%) under constant stirring at room temperature until pH 4.5 is reached. A typical foaming behavior and increase in temperature to 40 °C is observed. After 10 minutes, 102.7 g of the resulting slurry are directly transferred into a suitable autoclave and after sealing the reaction mixture is constantly stirred and heated to 170 °C. Upon reaching the desired internal temperature (around 6 bar pressure), the reaction is stirred for further 1 hour, before being cooled to 70 °C within 50 minutes. The resulting slurry is filtered and the obtained solid washed with 30.0 g of distilled water. The solid is dried over night at 50 °C to yield 18.3 g of Met-DKP in 77% yield.

### Example 7: Acidification of methionine side stream solution by pH shift with formic acid and isolation of Met-DKP

100 g of methionine side stream solution (3.2 wt.-% Met) containing 3.7 wt.-% MetMet (1.9 wt.-% DDLL-MetMet, 1.8 wt.-% DLLD-MetMet) with a measured pH value of 9.9 and a total K-content of 97.4 g/kg is acidified by slow addition of formic acid (9.7 g, 99%) under constant stirring at room temperature until pH 5.5 is reached. A reduced foaming behavior and no significant increase in temperature to 24 °C is observed. After 10 minutes, 103.4 g of the almost clear solution are directly transferred into a suitable autoclave and after sealing the reaction mixture is constantly stirred and heated to 160 °C. Upon reaching the desired internal temperature (around 5 bar pressure), the reaction mixture is stirred for further 2.5 hours, before being cooled to 70 °C within 40 minutes. The resulting slurry is filtered and the obtained solid washed with 25.0 g of distilled water. The solid is dried over night at 50 °C to yield 1.7 g of Met-DKP in 43% yield (88% purity).

### Example 8: Acidification of methionine side stream solution by pH shift with phosphoric acid and isolation of Met-DKP

100 g of methionine side stream solution (3.0 wt.-% Met) containing 3.3 wt.-% MetMet (1.6 wt.-% DDLL-MetMet, 1.7 wt.-% DLLD-MetMet) with a measured pH value of 8.3 and a total K-content of 93.1 g/kg is acidified by slow addition of phosphoric acid (19.9 g, 85%) under constant stirring at room temperature until pH 5.0 is reached. A typical foaming behavior and increase in temperature to 32 °C is observed. After 10 minutes, 112.1 g of the resulting slurry are directly transferred into a suitable autoclave and after sealing the reaction mixture is constantly stirred and heated to 160 °C. Upon reaching the desired internal temperature (around 5 bar pressure), the reaction is stirred for further 2.5 hours, before being cooled to 70 °C within 40 minutes. The resulting slurry is filtered and the obtained solid washed with 28.2 g of distilled water. The solid is dried over night at 50 °C to yield 2.9 g of Met-DKP in 75% yield (80% purity).

### Example 9: Acidification of methionine side stream solution by pH shift with diluted sulfuric acid and isolation of Met-DKP

100 g of methionine side stream solution (3.0 wt.-% Met) containing 3.3 wt.-% MetMet (1.6 wt.-% DDLL-MetMet, 1.7 wt.-% DLLD-MetMet) with a measured pH value of 8.3 and a total K-content of 93.1 g/kg is acidified by slow addition of diluted sulfuric acid (28.5 g, 30%) under constant stirring at room temperature until pH 5.5 is reached. A reduced foaming behavior and increase in temperature to 28 °C is observed. After 10 minutes, 121.7 g of the resulting slurry are directly transferred into a suitable and sealed autoclave and the reaction mixture is constantly stirred and heated to 160 °C. Upon reaching the desired internal temperature (around 5 bar pressure), the reaction is stirred for further 2.5 hours, before being cooled to 70 °C within 45 minutes. The resulting slurry is filtered and the obtained solid washed with 27.0 g of distilled water. The solid is dried over night at 50 °C to yield 4.9 g of Met-DKP in 64% yield (40% purity).

### Example 10: Acidification of methionine side stream solution by pH shift with sulfuric acid and isolation of Met-DKP

100 g of methionine side stream solution (4.1 wt.-% Met) containing 3.9 wt.-% MetMet (1.8 wt.-% DDLL-MetMet, 2.1 wt.-% DLLD-MetMet) with a measured pH value of 9.4 and a total K-content of 103.2 g/kg is acidified by slow addition of conc. sulfuric acid (3.6 g, 96%) under constant stirring at room temperature until pH 8.0 is reached. A reduced foaming behavior and increase in temperature to 31 °C is observed. After 10 minutes, 100.5 g of the resulting slurry are directly transferred into a suitable autoclave and after sealing the reaction mixture is constantly stirred and heated to 160 °C. Upon reaching the desired internal temperature (around 7 bar pressure), the reaction mixture is stirred for further 2.5 hours, before being cooled to 70 °C within 45 minutes. The resulting slurry is filtered and the obtained solid washed with 27.5 g of distilled water. The solid is dried over night at 50 °C to yield 2.0 g of an undefined solid ( <1 wt.-% Met-DKP yield). HPLC analysis revealed that only about 6 wt.-% MetMet was converted into Met-DKP.

### Example 11: Attempt of direct usage of methionine side stream solution for the isolation of Met-DKP

100 g of methionine side stream solution (3.2 wt.-% Met) containing 3.4 wt.-% MetMet (1.6 wt.-% DDLL-MetMet, 1.8 wt.-% DLLD-MetMet) with a measured pH value of 8.4 and a total K-content of 88.8 g/kg is directly transferred into a suitable autoclave and after sealing the reaction mixture is constantly stirred and heated to 160 °C. Upon reaching the desired internal temperature (around 7 bar pressure), the reaction mixture is stirred for further 2.5 hours, before being cooled to 70 °C within 30 minutes. The resulting slurry is filtered and the obtained solid is attempted to be washed with 23.7 g of distilled water. Unfortunately, after the initial washing step, no solid remained for drying and analysis. HPLC analysis revealed that only about 1 wt.-% MetMet was converted into Met-DKP.

### Analytical Methods

### High pressure liquid chromatography (HPLC):

Quantification of D,L-Met, DDLL-MetMet, DLLD-MetMet, DDLL-Met-DKP and DLLD-Met-DKP was performed after calibration with the respective reference substances using an isocratic HPLC method. All analyses were performed on an Agilent 1260 Infinity II device with HPLC pump G7112B, VWD detector G7114A and column thermostat G7116A. A standard RP 18 column was used for separation (Altima C18, 5 mm, 25 cm length, 40 °C), running an eluent system consisting of 900 g deionized water, 70 g acetonitrile and 40 g phosphoric acid (85%) at a flow rate of 1 ml/min and elution time of 35 minutes. The detection wavelength was set to 210 nm. For sample preparation, 20-100 mg of the were weighed into a volumetric flask that was filled with the eluent system to precisely 100 ml and degassed in an ultrasonic bath prior to injection.

### Ion Chromatography (IC):

The total potassium content (total K-content) within the initial aqueous potassium containing media was measured by IC after calibration with a reference substance. All analyses were performed on a Metrohm 882 Compakt IC Plus system equipped with a Methrom 850 Professional IC Detektor LF unit and a Metrohm Metrosep C6 250/4.0 column and Methrom Metrosep C6 Guard/4.0 precolumn. A pure aqueous eluent system containing 1.7 mmol/l nitric acid, 1.7 mmol/l dipicolinic acid and 2.5% (v/v) acetonitrile was used running the system at room temperature with a flow rate of 1 ml/min. For sample preparation, an appropriate amount of the substance was weighed into a volumetric flask that was filled with 2.5 mmol/l nitric acid solution to precisely 100 ml and degassed in an ultrasonic bath prior to injection.

## Claims

1. Method of recovery of methionylmethionine from an aqueous alkali metal ions containing medium, **characterized in that** methionylmethionine as contained in the aqueous medium is converted into Bis(methionyl)-diketopiperazine.

2. Method according to claim 1, **characterized in that** for the conversion of methionylmethionine into Bis(methionyl)-diketopiperazine the medium is heated in a sealed pressure vessel to a temperature of 120 to 200 °C, in particular 150 to 180 °C, preferably 155 to 175 °C, more preferably 160 to 170 °C.

3. Method according to claim 2, **characterized in that** the reaction is carried out at a pressure of 2 to 12 bar, preferably 4 to 10 bar.

4. Method according to any of the preceding claims, **characterized in that** the medium has a pH, measured with a pH electrode at 20°C, of 3 to 7, more preferably 3.5 to 6, in particular 4.0 to 5.5.

5. Method according to claim 4, **characterized in that** the starting medium has a higher pH, preferably a pH, measured with a pH electrode at 20°C, of 7.5 to 12.5, more preferably of 8 to 10, and adjusting the pH of 3 to 7, preferably 3.5 to 6, more preferably 4.0 to 5.5, is carried out by acidification of the starting medium, wherein acidification is preferably carried out by using an acid selected from phosphoric acid, acetic acid, formic acid, sulfuric acid or mixtures thereof.

6. Method according to any of the preceding claims, wherein the alkali metal ions are selected from potassium ions and sodium ions and mixtures thereof, but are preferably potassium ions.

7. Method according to any of the preceding claims, wherein the aqueous medium contains methionylmethionine in an amount of 1 to 12 wt.-%, preferably in an amount of 2 to 8 wt.-%, more preferably in an amount of 3 to 7 wt.-%.

8. Method according to any of the preceding claims, wherein the aqueous medium contains alkali metal ions, preferably potassium ions, in an amount of at least 1 wt.-%, in particular in an amount of 2 to 18 wt.-%, preferably in an amount of at least 4 wt.-%, in particular in an amount of 4 to 16 wt.-%, more preferably in an amount of at least 6 wt.-%, in particular in an amount of 6 to 14 wt.-%.

9. Method according to any of the preceding claims, wherein the aqueous medium contains methionine, preferably in an amount of 1 to 10 wt.-%, more preferably in an amount of 2 to 6 wt.-%.

10. Method according to any of the preceding claims, wherein the aqueous medium contains hydrogencarbonate, preferably in an amount of at least 1 wt.-%, in particular in an amount of 1 to 15 wt.-%, more preferably in an amount of at least 2 wt.-%, in particular in an amount of 2 to 12 wt.-%, above all in an amount of at least 3 wt.-%, in particular in an amount of 3 to 10 wt.-%.

11. Method according to any of the preceding claims, wherein the aqueous medium is a complex matrix, in particular a by-product of a chemical methionine production process.

12. Method according to any of the previous claims, wherein before the conversion of methionylmethionine into Bis(methionyl)-diketopiperazine by reaction in a sealed pressure vessel the major part of the alkali metal ions as contained in the medium is separated by formation of an insoluble alkali metal salt, preferably by adjusting a pH of 3 to 7, more preferably of 4 to 5, with sulfuric acid, and subsequent separation of the potassium salt thus obtained.

13. Method according to any of the preceding claims, wherein the Bis(methionyl)-diketopiperazine, as formed in the reaction, is crystallized or precipitated and then separated, preferably by filtration, centrifugation and/or decantation.

14. Method according to any of the preceding claims, wherein the Bis(methionyl)-diketopiperazine, after separation from the aqueous medium, is converted into methionylmethionine again by basic or acidic hydrolysis.

15. Method according to any of the claims 1 to 13, wherein the Bis(methionyl)-diketopiperazine, after separation from the aqueous solution, is converted into methionine by basic or acidic hydrolysis.
